# EUROPÄISCHE PATENTSCHRIFT

(11) **EP 2 707 492 B1**
(45) Veröffentlichungstag und Bekanntmachung des Hinweises auf die Patenterteilung: **31.12.2014**
(21) Anmeldenummer: 12717783.0
(22) Anmeldetag: 04.05.2012
(51) Int. Cl.: C12P 13/12

(54) **Verfahren zur fermentativen Herstellung von L-Cystin bei kontrollierter Sauerstoffsättigung**
Process for producing L-cystine by fermentation at controlled oxygen saturation
Procédé de préparation de L-cystine par fermentation à saturation en oxygène contrôlée

(30) Priorität: 11.05.2011 DE 102011075656
(43) Veröffentlichungstag der Anmeldung: 19.03.2014
(73) Patentinhaber: Wacker Chemie AG, 81737 München (DE)
(72) Erfinder: DASSLER, Tobias, 81825 München (DE); REUTTER-MAIER, Anneliese, 85614 Kirchseeon (DE); SCHLÖSSER, Thomas, 84489 Burghausen (DE)
(74) Vertreter: Potten, Holger
(86) Internationale Anmeldenummer: PCT/EP2012/058175
(87) Internationale Veröffentlichungsnummer: WO 2012/152664

(56) Entgegenhaltungen:
- EP-A1- 0 885 962
- EP-A2- 1 496 111
- KUMON, S. ET AL.: "Simultaneous oxidative reaction crystallization of L-cystine from L-cysteine with enzyme reactions of DL-amino-thiazoline-carboxylic acid as feed material", FOOD AND BIOPRODUCTS PROCESSING: TRANSACTIONS OF THE INSTITUTION OF CHEMICAL ENGINEERS, Bd. 72, Nr. 2, 1994, Seiten 86-90, XP008153052,

## Beschreibung

Die Erfindung betrifft ein Verfahren zur fermentativen Produktion von L-Cystin.

L-Cystin ist ein Disulfid, das bei der Oxidation von zwei Molekülen L-Cystein entsteht. Diese Reaktion ist reversibel, was bedeutet, dass L-Cystin durch Reduktion wieder in L-Cystein überführt werden kann.

Die Aminosäure L-Cystein ist von wirtschaftlicher Bedeutung. Sie wird beispielsweise als Lebensmittelzusatzstoff (insbesondere in der Backmittelindustrie), als Einsatzstoff in der Kosmetik, sowie als Ausgangsprodukt für die Herstellung von Pharmawirkstoffen (insbesondere N-Acetyl-Cystein und S-CarboxyMethyl-Cystein) verwendet.

L-Cystein nimmt in allen Organismen eine Schlüsselposition im Schwefelmetabolismus ein und wird in der Synthese von Proteinen, Glutathion, Biotin, Liponsäure, Methionin und anderen schwefelhaltigen Metaboliten verwendet. Zudem dient L-Cystein als Vorläufer für die Biosynthese von Coenzym A. Die Biosynthese von L-Cystein wurde in Bakterien, insbesondere in Enterobakterien, detailliert untersucht und ist ausführlich in Kredich (1996, Biosynthesis of cysteine, p. 514-527. *In* F. C. Neidhardt, R. Curtiss III, J. L. Ingraham, E. C. C. Lin, K. B. Low, B. Magasanik, W. S. Reznikoff, M. Riley, M. Schaechter, and H. E. Umbarger (ed.), Escherichia coli and Salmonella: cellular and molecular biology, 2nd ed. ASM Press, Washington, D.C.) beschrieben.

Neben der klassischen Gewinnung von L-Cystein mittels Extraktion aus keratinhaltigem Material wie Haaren, Borsten, Hörnern, Hufen und Federn oder mittels Biotransformation durch enzymatische Umsetzung von Vorstufen, beschrieben in KUMON, S. ET AL.: "Simultaneous oxidative reaction crystallization of L-cystine from L-cysteine with enzyme reactions of DL-amino-thiazoline-carboxylic acid as feed material" (FOOD AND BIOPRODUCTS PROCESSING: TRANSACTIONS OF THE INSTITUTION OF CHEMICAL ENGINEERS, Bd. 72, Nr. 2, 1994, Seiten 86-90), wurde vor einigen Jahren auch ein Verfahren zur fermentativen Herstellung von L-Cystein entwickelt. Der Stand der Technik bezüglich der fermentativen Gewinnung von L-Cystein mit Mikroorganismen ist ausführlich z.B. in US6218168B1, US5972663A, US2004/0038352A1, CA2386539A1, US2009/0053778A1 und US2009/0226984A1 beschrieben. Als bakterielle Wirtsorganismen werden dabei u.a. Stämme der Gattung Corynebakterium sowie Vertreter aus der Familie der Enterobacteriaceae, wie z.B. *Escherichia coli* oder *Pantoea ananatis* verwendet.

Neben der klassischen Vorgehensweise, um durch Mutation und Selektion zu verbesserten L-Cystein-Produzenten zu gelangen, wurden auch gezielt genetische Veränderungen an den Stämmen vorgenommen, um eine effektive L-Cystein-Überproduktion zu erreichen.

So führte das Einbringen eines cysE-Allels, das für eine Serin-O-Acetyl-Transferase mit einer verminderten Feedback-Hemmung durch L-Cystein kodiert, zu einer Steigerung der Cystein-Produktion (US6218168B1; Nakamori et al., 1998, Appl. Env. Microbiol. 64: 1607-1611; Takagi et al., 1999, FEBS Lett. 452: 323-327). Durch ein feedback-resistentes CysE-Enzym wird die Bildung von O-Acetyl-L-Serin, der direkten Vorstufe von L-Cystein, weitgehend vom L-Cystein-Spiegel der Zelle entkoppelt.

O-Acetyl-L-Serin wird aus L-Serin und Acetyl-CoA gebildet. Daher ist die Bereitstellung von L-Serin in ausreichender Menge für die L-Cystein-Produktion von großer Bedeutung. Dies kann durch das Einbringen eines serA-Allels, das für eine 3-Phosphoglycerat-Dehydrogenase mit einer verminderten Feedback-Hemmbarkeit durch L-Serin kodiert, erreicht werden. Dadurch wird die Bildung von 3-Hydroxypyruvat, einer Vorstufe von L-Serin, weitgehend vom L-Serin-Spiegel der Zelle entkoppelt. Beispiele für derartige SerA-Enzyme sind in EP0620853, US 2005/0009162 A1, US2005009162A2 und EP0931833 beschrieben.

Darüber hinaus ist bekannt, dass die L-Cystein-Ausbeute in der Fermentation dadurch erhöht werden kann, dass Gene abgeschwächt oder zerstört werden, die für L-Cystein-abbauende Enzyme kodieren, wie z.B. die Tryptophanase TnaA oder die Cystathionine-β-Lyasen MalY oder MetC (EP1571223).

Die Steigerung des Transports von L-Cystein aus der Zelle ist eine weitere Möglichkeit, um die Produkt-Ausbeute im Medium zu erhöhen. Dies kann durch Überexpression sogenannter EffluxGene erreicht werden. Diese Gene kodieren für membrangebundene Proteine, die den Export von L-Cystein aus der Zelle vermitteln. Verschiedene Effluxgene für den L-Cystein-Export wurden beschrieben (US5972663A, US2004/0038352A1, US2005221453, WO2004113373).

Der Export von L-Cystein aus der Zelle in das Fermentationsmedium hat mehrere Vorteile:
1) L-Cystein wird kontinuierlich aus dem intrazellulären Reaktionsgleichgewicht entzogen mit der Folge, dass der Spiegel dieser Aminosäure in der Zelle niedrig gehalten wird und somit die Feedback-Inhibiton von sensitiven Enzymen durch L-Cystein unterbleibt:
   (1) L-Cystein (intrazellulär) ⇆ L-Cystein (Medium)
2) Das in das Medium ausgeschiedene L-Cystein wird in Gegenwart von Sauerstoff, der während der Kultivierung in das Medium eingebracht wird, zum Disulfid L-Cystin oxidiert (US5972663A):
   (2) 2 L-Cystein + 1/2 O₂ ⇆ L-Cystin + H₂O

   Da die Löslichkeit von L-Cystin in wässriger Lösung bei einem neutralen pH-Wert v.a. im Vergleich zu L-Cystein nur sehr gering ist, fällt das Disulfid schon bei einer niedrigen Konzentration aus und bildet einen weißen Niederschlag:
   (3) L-Cystin (gelöst) ⇆ L-Cystin (Niederschlag)

   Durch die Präzipitation von L-Cystin wird der Spiegel des im Medium gelösten Produkts abgesenkt, wodurch auch jeweils das Reaktionsgleichgewicht von (1) und (2) auf die Produktseite gezogen wird.
3) Der technische Aufwand für die Reinigung des Produkts ist bedeutend geringer, wenn die Aminosäure direkt aus dem Fermentationsmedium gewonnen werden kann, als wenn das Produkt intrazellulär akkumuliert und zuerst ein Zellaufschluß erfolgen muss.

Fakultativ anaerobe Bakterien, wie z.B. *E. coli* oder *P. ananatis,* wachsen besser unter aeroben Bedingungen, d.h. in Gegenwart von Sauerstoff, da sie über den aeroben Stoffwechsel mehr Energie aus Kohlenhydraten wie z.B. Glucose gewinnen können, als durch die Vergärung (anaerober Metabolismus) der jeweiligen Energiequelle.

Auch die L-Cystein-Produktion mit *E. coli* oder *P. ananatis* wird unter aeroben Bedingungen durchgeführt (US2004/0038352A1, US5972663A, CA2386539A1, EP1389427, EP2138585). Bei den detaillierter beschriebenen Verfahren ist offenbart, dass der Sauerstoff-Eintrag in die Kultur so eingestellt wird, dass die Kulturbrühe während der Fermentation einen Sauerstoff-Gehalt von 50% aufweist.

Ein Nachteil bei den beschriebenen Verfahren zur fermentativen L-Cystein-Produktion ist, dass die Aminosäure in der Kulturbrühe in verschiedenen Formen vorliegt. Neben dem präzipitierten L-Cystin im Niederschlag findet man im Kulturüberstand L-Cystin in gelöster Form sowie L-Cystein und auch ein Thiazolidin (US6218168B1, US5972663A, CA2386539A1). Dieses Thiazolidin (2-Methyl-thiazolidin-2,4-dicarbonsäure) stellt das Kondensationsprodukt aus L-Cystein und Pyruvat dar, das in einer rein chemischen Reaktion gebildet wird.

Unter dem Begriff "Gesamtcystein" werden im Rahmen dieser Erfindung L-Cystein und die daraus gebildeten Verbindungen L-Cystin und Thiazolidin, die während der Fermentation entstehen und im Kulturüberstand und im Niederschlag akkumulieren, zusammengefasst.

Bei den bekannten Verfahren variiert am Ende der Fermentation der Anteil des präzipitierten L-Cystins. Dieser liegt zwischen 40-66% (US5972663A, CA2386539A1) des Gesamtcysteins, was bedeutet, dass die restlichen 34-60% des Gesamtcysteins im Kulturüberstand vorliegen und zwar überwiegend in Form von L-Cystein und Thiazolidin. Diese Produktheterogenität erschwert die Gewinnung und Reinigung des Produkts aus der Kulturbrühe.

Daher ist ein Verfahren wünschenswert, bei dem das Endprodukt größtenteils in nur einer Form anfällt. Besonders günstig ist ein Verfahren, bei dem als Produkt vorwiegend L-Cystin entsteht, denn aufgrund seiner geringen Löslichkeit in einem pH-neutralen wässrigen Medium fällt L-Cystin schon bei einer niedrigen Konzentration aus, wodurch das Reaktionsgleichgewicht auf die Produktseite verlagert wird, was wiederum zu höheren Produktausbeuten führt.

Aufgabe der vorliegenden Erfindung ist es demnach, ein Verfahren zur Herstellung von L-Cystin mittels Fermentation eines Mikroorganismenstammes in einem Fermentationsmedium zur Verfügung zu stellen, bei dem L-Cystin in einer Menge von mindestens 70 % bezogen auf das gebildete Gesamtcystein präzipitiert.

Die Aufgabe wird gelöst durch ein Verfahren, welches dadurch gekennzeichnet ist, dass die O₂-Sättigung des Fermentationsmediums während der L-Cystin-Bildung bei minimal 1 % und maximal 40 ±3 % gehalten wird.

Die O₂-Sättigung des Mediums ist definiert als prozentualer Anteil der gelösten Sauerstoff-Menge an der maximal löslichen Sauerstoff-Menge unter den gegebenen Bedingungen.

Überraschenderweise zeigte sich, dass der präzipitierte L-Cystin-Anteil am Gesamtcystein in der Kulturbrühe am Ende der Fermentation bei mindestens 70 % (Gewicht pro Liter präzipitiertes L-Cystin zu Gewicht pro Liter Gesamtcystein) liegt, wenn die O₂-Sättigung in diesem niedrigen Bereich gehalten wird. Dieser Befund ist besonders deshalb erstaunlich, da in EP1389427 beschrieben wird, dass ein hoher L-Cystin-Anteil gerade dadurch erreicht werden kann, dass ein Oxidationsmittel, z.B. in Form von Sauerstoff oder Wasserstoffperoxid, verstärkt in die Kultur eingebracht wird.

Das erfindungsgemäße Verfahren ist auch deshalb sehr vorteilhaft, da die Reinigung des Produkts leicht möglich ist, denn das als Präzipitat vorliegende L-Cystin kann durch einen einfachen Dekanterschritt von den Zellen abgetrennt werden.

Die Cystin-Bildungsphase des erfindungsgemäßen Fermentationsverfahrens beginnt mit dem Zeitpunkt, ab dem erstmals L-Cystin als Niederschlag in der Kulturbrühe nachgewiesen werden kann und dauert bis zum Ende der Kultivierung an. Typischerweise beginnt diese Phase ca. 8-10 h nach Inokulation des Produktionsfermenters.

Als Mikroorganismen für das erfindungsgemäße Verfahren können alle im Stand der Technik beschriebenen Cystein-produzierenden Enterobacteriaceae Stämme eingesetzt werden. Derartige Stämme sind beispielsweise offenbart in US6218168B1, US5972663A, U82004/0038352A1, CA2386539A1, US2009/0053778 oder EP2138585.

Als Mikroorganismen bevorzugt sind Vertreter aus der Familie der Enterobacteriaceae, besonders bevorzugt Vertreter der Gattungen *Escherichia* und *Pantoea,* ganz besonders bevorzugt sind Stämme der Spezies *E. coli* und *P. ananatis.*

Von diesen Mikroorganismenstämmen sind wiederum Stämme bevorzugt, die entweder eine veränderte Serin-O-Acetyl-Transferase besitzen, die im Vergleich zu dem entsprechenden Wildtypenzym eine um mindestens den Faktor 2 verminderte Feedback-Hemmung durch L-Cystein aufweist, oder die durch Überexpression eines Effluxgens einen um mindestens den Faktor 2 erhöhten Cystein-Export aus der Zelle im Vergleich zu einer Wildtypzelle aufweisen. Besonders bevorzugte Mikroorganismenstämme besitzen sowohl eine Serin-O-Acetyl-Transferase, die im Vergleich zu dem entsprechenden Wildtypenzym eine um mindestens den Faktor 2 verminderte Feedback-Hemmung durch L-Cystein aufweist, als auch einen durch Überexpression eines Effluxgens um mindestens den Faktor 2 erhöhten Cystein-Export aus der Zelle im Vergleich zu einer Wildtypzelle. Derartige Stämme sind beispielsweise aus US6218168B1 und US5972663A bekannt. Ganz besonders bevorzugte Stämme sind solche, die zusätzlich eine veränderte 3-Phosphoglycerat-Dehydrogenase mit einer im Vergleich zu dem entsprechenden Wildtypenzym um mindestens den Faktor 2 verminderten Feedback-Hemmung durch L-Serin besitzen (US 2005/0009162A1, US2005009162A2) und bei denen mindestens ein L-Cystein-abbauendes Enzym soweit abgeschwächt ist, dass in der Zelle nur noch maximal 50 % dieser Enzym-Aktivität im Vergleich zu einer Wildtypzelle vorhanden ist.

Bevorzugte Varianten der Serin-O-Acetyl-Transferase weisen im Vergleich zu dem entsprechenden Wildtypenzym eine um mildestens den Faktor 5, besonders bevorzugt um mindestens den Faktor 10, ganz besonders bevorzugt um mindestens den Faktor 50 verminderte Feedback-Hemmung durch L-Cystein auf.

Das Effluxgen stammt vorzugsweise aus der Gruppe ydeD (siehe US5972663A), yfiK (siehe US2004/0038352A1), cydDC (siehe WO2004113373), bcr (siehe US2005221453) und emrAB (siehe US2005221453) von E. coli oder dem entsprechenden homologen Gen aus einem anderen Mikroorganismus. Unter einem homologen Gen ist zu verstehen, dass die Sequenz dieses Gens zu mindestens 80 % mit der DNA-Sequenz des entsprechenden E. coli-Gens übereinstimmt.

Die Überexpression eines Effluxgens führt im Vergleich zu einer Wildtypzelle bevorzugt zu einem um mindestens den Faktor 5, besonders bevorzugt um mindestens den Faktor 10, besonders bevorzugt um mindestens den Faktor 20 erhöhten Cystein-Export aus der Zelle.

Bevorzugte Varianten der 3-Phosphoglycerat-Dehydrogenase weisen im Vergleich zu dem entsprechenden Wildtypenzym eine um mindestens den Faktor 5, besonders bevorzugt um mindestens den Faktor 10, ganz besonders bevorzugt um mindestens den Faktor 50 verminderte Feedback-Hemmung durch L-Serin auf.

Das L-Cystein-abbauende Enzym stammt vorzugsweise aus der Gruppe Tryptophanase (TnaA) und Cystathionine-β-Lyase (MalY, MetC).

Besonders bevorzugt sind Mikroorganismenstämme, in denen mindestens eines dieser Enzyme soweit abgeschwächt ist, dass in der Zelle nur noch maximal 10 % der Enzym-Aktivität im Vergleich zu einem Wildtypstamm vorhanden ist. Ganz besonders bevorzugt sind Stämme, in denen mindestens eines dieser Enzyme völlig inaktiviert ist.

Die Kultivierung der Zellen bei der L-Cystein-Produktion wird unter aeroben Wachstumsbedingungen durchgeführt. Der Sauerstoff kann auf unterschiedliche Weise in die Kultur eingebracht werden, so z.B. durch Schütteln des Kulturgefäßes mittels einer Schüttelvorrichtung. Erfolgt die Kultivierung in einem Fermenter, kann entweder Luft oder reiner Sauerstoff oder ein Gemisch dieser Gase in die Kultur geblasen werden. Um eine ausreichende Sauerstoff-Versorgung der Zellen zu gewährleisten, kann die Fermentation auch unter Druck erfolgen (z.B. bei 0,5-1,2 bar Überdruck), wodurch die Löslichkeit von Sauerstoff im Medium gesteigert wird. Bei einem Rührkesselfermenter wird der eingebrachte Sauerstoff zusätzlich durch ein Rührwerk im Medium dispergiert, wodurch die Verfügbarkeit des Sauerstoffs für die Zellen erhöht wird.

Vorzugsweise wird während der Fermentation der im Medium gelöste Sauerstoff ständig mittels einer Sonde gemessen. Dies kann z.B. mit einem optischen Sensor oder einem chemischen Sensor (Clark-Zelle) erfolgen. Die ermittelte O₂-Sättigung des Mediums wird laufend mit dem vorgegebenen Soll-Wert abgeglichen. Weicht der Ist-Wert vom Soll-Wert ab, wird über einen automatischen Regelkreis entweder die Gaszufuhr oder die Rührgeschwindigkeit oder beides derart angepasst, dass die O₂-Sättigung wieder an den Soll-Wert herangeführt wird. Auf diese Weise kann der Sauerstoff-Gehalt in der Kultur über die gesamte Fermentationsdauer bei einem gewünschten Wert gehalten werden.

Die Sauerstoff-Meßsonde wird vor Beginn der Fermentation auf die Grenzwerte 0% und 100% O₂-Sättigung im noch unbeimpften Medium kalibriert. Dies geschieht sinnvollerweise bei der späteren Fermentationstemperatur.

Bevor die Sonde auf 0% O₂-Sättigung kalibriert werden kann, muss zuerst der gesamte noch vorhandene Sauerstoff aus dem Medium ausgetrieben werden. Dies geschieht automatisch bei der *in situ*-Sterilisation des Fermentationsmediums im Fermenter oder kann durch Begasung des Mediums mit reinem Stickstoff bei einer Rührgeschwindigkeit von 20 % der maximal möglichen Rührgeschwindigkeit erfolgen. Die Kalibrierung der Sonde auf 100% O₂-Sättigung erfolgt in der Regel unter den Sauerstoff-Versorgungsbedingungen, wie sie bei Beginn der Fermentation vorliegen (Startbedingungen), d.h. bei 40 % der maximal möglichen Gaszufuhr und bei 20 % der maximal möglichen Rührgeschwindigkeit. Die Kalibrierung der Sonde auf 100% erfolgt erst dann, wenn der Sauerstoff-Gehalt unter den gewählten Bedingungen einen konstanten Wert erreicht hat.

Der Sauerstoff-Gehalt während der Produktionsphase der L-Cystin-Fermentation muss unter 40 ±3 % O₂-Sättigung, bevorzugt unter 30 ±3 %, besonders bevorzugt unter 20 ±3 %, ganz besonders bevorzugt unter 10 ±3 % und bei minimal 1% gehalten werden.

Als Kohlenstoffquelle dienen vorzugsweise Zucker, Zuckeralkohole, organische Säuren oder zuckerhaltige Pflanzenhydrolysate. Besonders bevorzugt werden im erfindungsgemäßen Verfahren als Kohlenstoffquelle Glucose, Fructose, Lactose, Glycerin oder Gemische, die zwei oder mehr dieser Verbindungen enthalten, eingesetzt.

Bevorzugt wird die Kohlenstoffquelle der Kultur so zudosiert, dass der Gehalt der Kohlenstoffquelle im Fermenter während der L-Cystein-Produktionsphase 10 g/l nicht übersteigt. Bevorzugt ist eine maximale Konzentration von 2 g/l, besonders bevorzugt von 0,5 g/l, ganz besonders bevorzugt von 0,1 g/l.

Als N-Quelle werden im erfindungsgemäßen Verfahren vorzugsweise Ammoniak, Ammoniumsalze oder Proteinhydrolysate verwendet. Bei Verwendung von Ammoniak als Korrekturmittel zur pH-Statisierung wird während der Fermentation regelmäßig diese N-Quelle nachdosiert.

Als weitere Medienzusätze können Salze der Elemente Phosphor, Chlor, Natrium, Magnesium, Stickstoff, Kalium, Calcium, Eisen und in Spuren (d.h. in µM Konzentrationen) Salze der Elemente Molybdän, Bor, Kobalt, Mangan, Zink und Nickel zugesetzt werden.

Des Weiteren können organische Säuren (z.B. Acetat, Citrat), Aminosäuren (z.B. Isoleucin) und Vitamine (z.B. B1, B6) dem Medium zugesetzt werden.

Als komplexe Nährstoffquellen können z.B. Hefeextrakt, Maisquellwasser, Sojamehl oder Malzextrakt zum Einsatz kommen.

Die Inkubationstemperatur für mesophile Mikroorganismen wie z.B. *E. coli* öder *P*. *ananatis* beträgt vorzugsweise 15 - 45 °C, besonders bevorzugt 30 - 37 °C.

Der pH-Wert des Fermentationsmediums liegt während der Fermentation bevorzugt im pH-Bereich von 5,0 bis 8,5, besonders bevorzugt ist ein pH-Wert von 7,0.

Für die Herstellung von L-Cystein und L-Cystein-Derivaten muss während der Fermentation eine Schwefelquelle zugefüttert werden. Bevorzugt kommen dabei Sulfate oder Thiosulfate zum Einsatz.

Mikroorganismen, die nach dem beschriebenen Verfahren fermentiert werden, sezernieren in einem Batch- oder Fedbatch-Prozess nach einer Anwachsphase in einem Zeitraum von acht bis 150 Stunden L-Cystein und davon abgeleitete Verbindungen in hoher Effizienz in das Fermentationsmedium.

Nach der Fermentation kann das als Niederschlag vorliegende L-Cystin nach bekannten Verfahren von den restlichen Bestandteilen der Kulturbrühe beispielsweise mit Hilfe eines Dekanters abgetrennt werden.

Zur weiteren Aufreinigung des Rohprodukts können folgende Schritte durchgeführt werden:
- Lösen des Rohproduktes mit einer Mineralsäure
- Klären der Rohproduktlösung durch Zentrifugation oder Filtration
- Entfärbung der Lösung
- Fällungskristallisation

Die Reduktion von L-Cystin zu L-Cystein kann beispielsweise über einen elektrochemischen Prozess, wie in EP0235908 beschrieben, erfolgen.

Die folgenden Beispiele dienen der weiteren Erläuterung der Erfindung.

### Beispiel 1: Erzeugung von Cystein-Produktionsstämmen

Es wurden die Wildtyp-Stämme *E. coli* W3110 (ATCC 27325) und *P. ananatis* (ATCC 11530) jeweils mit dem Plasmid pACYC184/cysEX-GAPDH-ORF306 (offenbart in Beispiel 2 von US5972663A) mittels Elektroporation wie in US5972663A beschrieben transformiert. Das Plasmid pACYC184/cysEX-GAPDH-ORF306 enthält neben dem Replikationsursprung und einem Tetracyclin-Resistenzgen auch noch das cysEX-Allel, das für eine Serin-O-Acetyl-Transferase mit einer verminderten Feedback-Hemmung durch L-Cystein kodiert sowie das Effluxgen ydeD (ORF306), dessen Expression durch den konstitutiven GAPDH-Promotor gesteuert wird.

Die Selektion auf Plasmid-tragende Zellen erfolgte auf LB-Agarplatten, die 15 mg/l Tetracyclin enthielten.

Nach einer erneuten Plasmidisolierung mittels dem QIAprep Spin Plasmid Kit (Qiagen GmbH) und einer Restriktionsanalyse wurden die gewünschten Transformanden, d.h. Zellen, die das Plasmid pACYC184/cysEX-GAPDH-ORF306 aufgenommen haben, isoliert und in der Fermentation eingesetzt, wie dies in Beispiel 2 beschrieben ist.

### Beispiel 2: Kultivierung der Cystein-Produktionsstämme bei unterschiedlicher Sauerstoff-Sättigung

### Vorkultur 1 (Schüttelkolben):

20 ml LB-Medium mit 15 mg/l Tetracyclin wurden in einem Erlenmeyerkolben (100 ml) mit dem jeweiligen Stamm (*E. coli* W3110 pACYC184/cysEX-GAPDH-ORF306 bzw. *P. ananatis* pACYC184/cysEX-GAPDH-ORF306) beimpft und für sieben Stunden auf einem Schüttler (150 rpm, 30 °C) inkubiert.

### Vorkultur 2 (Schüttelkolben):

Anschließend wurde die Vorkultur 1 vollständig in 100 ml SM1-Medium überführt (12 g/l K₂HPO₄, 3 g/l KH₂PO₄, 5 g/l (NH₄)₂SO₄, 0,3 g/l MgSO₄ x 7 H₂O, 0,015 g/l CaCl₂ x 2 H₂O, 0,002 g/l FeSO₄ x 7 H₂O, 1 g/l Na₃Citrat x 2 H₂O, 0,1 g/l NaCl, 1 ml/l Spurenelementlösung, bestehend aus 0,15 g/l Na₂MoO₄ x 2H₂O, 2,5 g/l H₃BO₃, 0,7 g/l CoCl₂ x 6 H₂O, 0,25 g/l CuSO₄ x 5 H₂O, 1,6 g/l MnCl₂ x 4 H₂O, 0,3 g/l ZnSO₄ x 7 H₂O), das mit 5 g/l Glucose, 5 mg/l Vitamin B1 und 15 mg/l Tetracyclin supplementiert war. Die Kulturen wurden in Erlenmeyerkolben (1 1) bei 30 °C für 17 h mit 150 rpm geschüttelt. Nach dieser Inkubation lag die optische Dichte bei 600 nm (OD₆₀₀) zwischen 3 und 5.

### Hauptkultur (Fermenter):

Die Fermentation wurde in Fermentern des Typs BIOSTAT B der Firma Sartorius Stedim durchgeführt. Es wurde ein Kulturgefäß mit 2 1 Gesamtvolumen verwendet. Das Fermentationsmedium (900 ml) enthält 15 g/l Glucose, 10 g/l Trypton (Difco), 5 g/l Hefeextrakt (Difco), 5 g/l (NH₄)₂SO₄, 1,5 g/l KH₂PO₄, 0,5 g/l NaCl, 0,3 g/l MgSO₄ x 7 H₂O, 0,015 g/l CaCl₂ x 2 H₂O, 0,075 g/l FeSO₄ x 7 H₂O, 1 g/l Na₃Citrat x 2 H₂O und 1 ml Spurenelementlösung (siehe oben), 0,005 g/l Vitamin B1 und 15 mg/l Tetracyclin.

Der pH-Wert im Fermenter wurde zu Beginn durch Zupumpen einer 25% NH₄OH-Lösung auf 7,0 eingestellt. Während der Fermentation wurde der pH-Wert durch automatische Korrektur mit 25% NH₄OH auf einem Wert von 7,0 gehalten. Zum Animpfen wurden 100 ml der Vorkultur 2 in das Fermentergefäß gepumpt. Das Anfangsvolumen betrug somit etwa 1 1. Die Kulturen wurden zu Beginn mit 400 rpm gerührt und mit 2 vvm einer über einen Sterilfilter entkeimten Druckluft begast. Unter diesen Startbedingungen war die Sauerstoff-Sonde vor der Inokulation auf 100% Sättigung kalibriert worden. Der Soll-Wert für die O₂-Sättigung während der Fermentation wurde - je nach Versuchsansatz - auf 50 ± 5%, 40 ± 5%, 30 ± 5%, 20 ± 5% oder 10 ± 5% eingestellt. Nach Absinken der O₂-Sättigung unter den jeweiligen Soll-Wert wurde eine Regulationskaskade gestartet, um die O₂-Sättigung wieder an den Soll-Wert heranzuführen. Dabei wurde zunächst die Gaszufuhr kontinuierlich erhöht (auf max, 5 vvm) und anschließend die Rührgeschwindigkeit (auf max. 1.500 rpm) kontinuierlich gesteigert.

Die Fermentation wurde bei einer Temperatur von 30 °C durchgeführt. Nach 2 h Fermentationszeit erfolgte die Zufütterung einer Schwefelquelle in Form einer sterilen 60 % Natrium-Thiosulfat x 5 H₂O - Stammlösung mit einer Rate von 1,5 ml pro Stunde. Sobald der Glukose-Gehalt im Fermenter von anfänglich 15 g/l auf ca. 2 g/l abgesunken war, erfolgte eine kontinuierliche Zudosierung einer 56% Glukose-Lösung. Die Fütterungsrate wurde so eingestellt, dass die Glukosekonzentration im Fermenter 2 g/l fortan nicht mehr überstieg. Die Glukose-Bestimmung wurde mit einem Glukoseanalysator der Firma YSI (Yellow Springs, Ohio, USA) durchgeführt.

Die Fermentationsdauer betrug 48 Stunden. Danach wurden Proben entnommen und der Gehalt von L-Cystein und den davon abgeleiteten Derivaten im Kulturüberstand (v.a. L-Cystein und Thiazolidin) und im Niederschlag (L-Cystin) jeweils getrennt voneinander bestimmt (s. Tabelle 1). Zu diesem Zweck wurde jeweils der colorimetrische Test von Gaitonde verwendet (Gaitonde, M. K. (1967), Biochem. J. 104, 627-633). Dabei ist zu beachten, daß unter den stark sauren Reaktionsbedingungen des Tests nicht nur freies L-Cystein, sondern auch das im Thiazolidin gebundene L-Cystein mit erfasst und quantifiziert wird. Im Kulturüberstand gelöstes L-Cystin wird im Test von Gaitonde nach Reduktion mit Dithiothreitol (DTT) in verdünnter Lösung bei pH 8,0 ebenfalls als L-Cystein nachgewiesen. Das im Niederschlag befindliche L-Cystin musste zuerst in 8% Salzsäure aufgelöst werden, bevor es auf dieselbe Art quantifiziert werden konnte.

**Tabelle 1: Gehalt von L-Cystein und L-Cystein-Derivaten in der Kulturbrühe nach 48 h**

| | ***E.coli*** | | | ***P.ananatis*** | | |
|---|---|---|---|---|---|---|
| **Sollwert O₂-Sättigung** | **Cystein**-**Gehalt [g/L]** | | **Anteil Cystin** | **Cystein-Gehalt [g/L]** | | **Anteil Cystin** |
| **[%]** | **nach 48 h** | | **[%]³** | **nach 48 h** | | **[%]³** |
| | Überstand¹ | Niederschlag² | | Überstand¹ | Niederschlag² | |
| **50 ± 3 (Vergleichsbsp.)** | 6,3 | 11,0 | 63,6 | 3,8 | 8,2 | 68,3 |
| **40 ± 3** | 5,1 | 12,3 | 70,7 | 3,7 | 9,1 | 71,1 |
| **30 ± 3** | 4,1 | 14,7 | 78,2 | 3,3 | 10,5 | 76,1 |
| **20 ± 3** | 2,7 | 16,4 | 85,9 | 2,6 | 12,8 | 83,1 |
| **10 ± 3** | 1,9 | 17,9 | 90,4 | 1,3 | 14,1 | 91,6 |

| | | | | | | |
|---|---|---|---|---|---|---|
| ¹: Summe des im Überstand gelösten L-Cysteins, L-Cystins und Thiazolidins ²: L-Cystin im Niederschlag ³: Anteil von L-Cystin im Niederschlag an Gesamtcystein | | | | | | |

## Patentansprüche

1. Verfahren zur Herstellung von L-Cystin durch Fermentation eines Mikroorganismenstammes in einem Fermentationsmedium, bei dem die Kultivierung der Zellen bei der L-Cystein-Produktion unter aeroben Wachstumsbedingungen durchgeführt wird und bei dem L-Cystin in einer Menge von mindestens 70 % bezogen auf Gesamtcystein präzipitiert, **dadurch gekennzeichnet, dass** als Mikroorganismenstamm ein Vertreter aus der Familie der Enterobacteriaceae eingesetzt wird und die O₂-Sättigung des Fermentationsmediums während der L-Cystin-Bildung bei minimal 1 % und maximal 40 ±3 % gehalten wird.

2. Verfahren nach Anspruch 1, **dadurch gekennzeichnet, dass** die O₂-Sättigung während der L-Cystin-Bildung unter 30 ±3 % gehalten wird.

3. Verfahren nach Anspruch 1 oder 2, **dadurch gekennzeichnet, dass** die O₂-Sättigung während der L-Cystin-Bildung unter 10 ±3 % gehalten wird.

4. Verfahren nach Anspruch 3 **dadurch gekennzeichnet, dass** der Mikroorganismenstamm entweder eine Serin-O-Acetyl-Transferase besitzt, die im Vergleich zu dem entsprechenden Wildtypenzym eine um mindestens den Faktor 2 verminderte Feedback-Hemmung durch L-Cystein aufweist, oder dass der Mikroorganismenstamm durch Überexpression eines Effluxgens einen um mindestens den Faktor 2 erhöhten Cystein-Export im Vergleich zu einem Wildtypstamm aufweist.

5. Verfahren nach Anspruch 4 **dadurch gekennzeichnet, dass** der Mikroorganismenstamm zusätzlich eine 3-Phosphoglycerat-Dehydrogenase mit einer im Vergleich zu dem entsprechenden Wildtypenzym um mindestens den Faktor 2 verminderten Feedback-Hemmung durch L-Serin aufweist.

6. Verfahren nach einem der Ansprüche 1 bis 5 **dadurch gekennzeichnet, dass** der Sauerstoff durch Schütteln des Kulturgefäßes mittels einer Schüttelvorrichtung oder durch Einblasen von Luft oder reinem Sauerstoff oder einem Gemisch dieser Gase in das Fermentationsmedium eingebracht wird.

7. Verfahren nach einem der Ansprüche 1 bis 6 **dadurch gekennzeichnet, dass** eine Kohlenstoffquelle dem Fermentationsmedium so zudosiert wird, dass der Gehalt der Kohlenstoffquelle im Fermenter während der Cystein-Produktionsphase 10 g/l nicht übersteigt.

8. Verfahren nach einem der Ansprüche 1 bis 7 **dadurch gekennzeichnet, dass** die Inkubationstemperatur 15 - 45 °C beträgt und der pH-Wert des Fermentationsmediums während der Fermentation bei einem pH-Wert im Bereich von 5,0 bis 8,5 liegt.

9. Verfahren nach einem der Ansprüche 1 bis 8 **dadurch gekennzeichnet, dass** während der Fermentation eine Schwefelquelle zugefüttert wird.

## Claims

1. Method for producing L-cystine by fermenting a microorganism strain in a fermentation medium, in which method the cultivation of the cells during the L-cysteine production is carried out under aerobic growth conditions and in which method L-cystine precipitates in an amount of at least 70% relative to the total cysteine, **characterized in that** the microorganism strain used is a representative from the Enterobacteriaceae family and the O₂ saturation of the fermentation medium during the formation of L-cystine is maintained at least at 1% and at most at 40 ± 3%.

2. Method according to Claim 1, **characterized in that** the O₂ saturation during the formation of L-cystine is maintained below 30 ± 3%.

3. Method according to Claim 1 or 2, **characterized in that** the O₂ saturation during the formation of L-cystine is maintained below 10 ± 3%.

4. Method according to Claim 3, **characterized in that** the microorganism strain either has a serine O-acetyltransferase, which has a feedback inhibition by L-cysteine reduced by at least a factor of 2 in comparison to the corresponding wild type enzyme, or that the microorganism strain has a cysteine export increased by at least a factor of 2 by overexpression of an efflux gene in comparison to a wild type strain.

5. Method according to Claim 4, **characterized in that** the microorganism strain additionally has a 3-phosphoglycerate dehydrogenase having a feedback inhibition by L-serine reduced by at least a factor of 2 in comparison to the corresponding wild type enzyme.

6. Method according to any of Claims 1 to 5, **characterized in that** the oxygen is introduced into the fermentation medium by shaking the culture vessel by means of a shaking device or by blowing in air or pure oxygen or a mixture of these gases.

7. Method according to any of Claims 1 to 6, **characterized in that** a carbon source is added to the fermentation medium such that the level of the carbon source in the fermenter during the cysteine production phase does not exceed 10 g/l.

8. Method according to any of Claims 1 to 7, **characterized in that** the incubation temperature is 15 - 45°C and the pH of the fermentation medium during the fermentation is at a pH in the range of 5.0 to 8.5.

9. Method according to any of Claims 1 to 8, **characterized in that** a sulfur source is fed in during the fermentation.

## Revendications

1. Procédé pour la préparation de L-cystine par fermentation d'une souche de microorganismes dans un milieu de fermentation, dans lequel la culture des cellules lors de la production de L-cystéine est réalisée dans des conditions de croissance aérobies et dans lequel la L-cystine précipite en une quantité d'au moins 70% par rapport à la cystéine totale, **caractérisé en ce qu'**on utilise comme souche de microorganismes un représentant de la famille des Enterobacteriaceae et on maintient la saturation en O₂ du milieu de fermentation pendant la formation de la L-cystine à au minimum 1% et au maximal 40 ± 3%.

2. Procédé selon la revendication 1, **caractérisé en ce que** la saturation en O₂ pendant la formation de la L-cystine est maintenue sous 30 ± 3%.

3. Procédé selon la revendication 1 ou 2, **caractérisé en ce que** la saturation en O₂ pendant la formation de la L-cystine est maintenue sous 10 ± 3%.

4. Procédé selon la revendication 3, **caractérisé en ce que** la souche de microorganismes possède soit une sérine-O-acétyl-transférase, qui présente une rétro-inhibition par la L-cystéine diminuée d'au moins un facteur 2 par rapport à l'enzyme de type sauvage correspondante, soit la souche de microorganismes présente une excrétion de cystéine augmentée d'au moins un facteur 2 par rapport à une souche de type sauvage par surexpression d'un gène d'efflux.

5. Procédé selon la revendication 4, **caractérisé en ce que** la souche de microorganismes présente en plus une 3-phosphoglycérate-déshydrogénase présentant une rétro-inhibition par la L-sérine diminuée d'au moins un facteur 2 par rapport à l'enzyme de type sauvage correspondante.

6. Procédé selon l'une quelconque des revendications 1 à 5, **caractérisé en ce que** l'oxygène est introduit dans le milieu de fermentation par agitation du récipient de culture au moyen d'un dispositif d'agitation ou par insufflation d'air ou d'oxygène pur ou d'un mélange de ces gaz.

7. Procédé selon l'une quelconque des revendications 1 à 6, **caractérisé en ce qu'**on ajoute en dosant au milieu de fermentation une source de carbone telle que la teneur en source de carbone dans le fermenteur ne dépasse pas 10 g/l pendant la phase de production de la cystéine.

8. Procédé selon l'une quelconque des revendications 1 à 7, **caractérisé en ce que** la température d'incubation est de 15-45°C et le pH du milieu de fermentation pendant la fermentation se situe à un pH dans la plage de 5,0 à 8,5.

9. Procédé selon l'une quelconque des revendications 1 à 8, **caractérisé en ce qu'**une source de soufre est alimentée pendant la fermentation.
